(19) **Europäisches Patentamt**

**European Patent Office**

**Office européen des brevets**

(11) **EP 0 996 449 B1**

(12) **EUROPEAN PATENT SPECIFICATION**

(45) Date of publication and mention
of the grant of the patent:
**20.02.2002 Bulletin 2002/08**

(51) Int Cl.⁷: **A61K 31/545**, A61K 9/14,
A61K 9/20

(21) Application number: **98940001.5**

(22) Date of filing: **07.08.1998**

(86) International application number:
**PCT/CA98/00773**

(87) International publication number:
**WO 99/08683 (25.02.1999 Gazette 1999/08)**

(54) **PHARMACEUTICAL COMPOSITIONS COMPRISING CEFUROXIME AXETIL**

ARZNEIZUBEREITUNGEN ENTHALTEND CEFUROXIM AXETIL

COMPOSITIONS PHARMACEUTIQUES CONTENANT UN CEFUROXIME AXETIL

(84) Designated Contracting States:
**AT BE CH CY DE DK ES FI FR GB GR IE IT LI LU MC NL PT SE**

(30) Priority: **15.08.1997 CA 2209868**

(43) Date of publication of application:
**03.05.2000 Bulletin 2000/18**

(73) Proprietor: **SHERMAN, Bernard Charles Willowdale, Ontario M2L 2K1 (CA)**

(72) Inventor: **SHERMAN, Bernard Charles Willowdale, Ontario M2L 2K1 (CA)**

(74) Representative: **Howard, Paul Nicholas et al Carpmaels & Ransford 43 Bloomsbury Square London WC1A 2RA (GB)**

(56) References cited:
**EP-A- 0 107 276       EP-A- 0 280 571**
**EP-A- 0 757 991       EP-A- 0 821 965**
**WO-A-98/22091        FR-A- 2 549 837**
**GB-A- 2 181 052       GB-A- 2 204 792**

## Description

BACKGROUND

Cefuroxime axetil is an antibiotic effective against a wide spectrum of microorganisms. Antibiotics for oral administration should be in a form which provides high bioavailability, whereby absorption into the bloodstream from the gastro-intestinal tract is maximized.

For cefuroxime axetil, the prior art discloses substantial difficulties in making compositions for oral administration providing high bioavailability.

Pure cefuroxime axetil can be produced in crystalline form or amorphous form. U.S. patent 4820833 discloses that the pure amorphous form is more soluble in water than the pure crystalline form and gives higher bioavailability upon oral administration.

U.S. patent 4897270 further discloses that film coated tablets comprising cefuroxime axetil (even in amorphous form) give low levels of absorption into the blood stream unless the tablets are formulated such that, when the tablet is ingested, the film coating ruptures very rapidly and the core then disintegrates immediately.

The prior art thus teaches that good absorption from tablets comprising cefuroxime axetil can be achieved only if the cefuroxime axetil used in the formulation is in pure amorphous form and the tablets contain sufficient disintegrant to cause them to disintegrate immediately in gastro-intestinal fluid.

It is the object of the present invention to overcome these limitations disclosed in the prior art.

More specifically, one object of the present invention is to enable compositions of cefuroxime axetil for oral administration exhibiting high bioavailability without requiring use of cefuroxime axetil in pure amorphous form; and a second object of the present invention is to enable tablets for oral administration exhibiting high bioavailability without requiring that the tablets disintegrate immediately in gastro-intestinal fluid.

BRIEF SUMMARY OF THE INVENTION

It has been found that the water solubility and hence bioavailability of cefuroxime axetil can be enhanced by making a co-precipitate comprising cefuroxime axetil and a water-soluble excipient.

It has further been found that tablets made from the co-precipitate exhibit satisfactory dissolution and bioavailability even if the tablets disintegrate over a period of many minutes, instead of immediately.

DETAILED DESCRIPTION OF THE INVENTION

As aforesaid, it has been found that the water-solubility of cefuroxime axetil can be enhanced by making a co-precipitate of cefuroxime axetil with a water-soluble excipient.

The term "water-soluble excipient" will be understood to mean an ingredient having no therapeutic activity and being nontoxic (and thus suitable as an excipient) that has a solubility in water of at least 1 g per 1000 g at 20°C. The solubility will preferably be at least 1 g per 100 g at 20°C, and more preferably at least 1 g per 10 g at 20°C. Suitable water-soluble excipients will include, for example, povidone, polyethylene glycols, hydroxypropyl cellulose, methylcellulose, lactose, mannitol and sorbitol.

A preferred water-soluble excipient is povidone. The amount of the water-soluble excipient used may be from about 2% to about 60% of the total weight of the co-precipitate, preferably from about 5% to about 25%, and most preferably about 10%.

The co-precipitate is made by dissolving pure crystalline cefuroxime axetil and the water-soluble excipient in a solvent or combination of solvents and evaporating the solvent or solvents. The solvent or solvents used will preferably be a solvent or solvents in which the cefuroxime axetil and the water soluble excipient have relatively high solubility so as to minimize the amount of solvent needed.

Since cefuroxime axetil has low solubility in water, it follows that a solvent other than water must be used to dissolve the cefuroxime axetil. Of the common organic solvents, the solvent in which cefuroxime axetil is most soluble is acetone. Acetone is thus a preferred solvent.

If the solvent selected to dissolve the cefuroxime axetil is also a good solvent for the water-soluble excipient, then only this one solvent is needed to dissolve both. However, if the solvent selected to dissolve the cefuroxime axetil is not a good solvent for the selected water-soluble excipient, then a second solvent is needed to dissolve the water-soluble excipient. That second solvent may be water or another organic solvent.

If two solvents are used, they should be capable of being inter-dissolved to enable formation of a clear solution of the cefuroxime axetil and the water-soluble excipient in the combination of solvents.

A solution of the cefuroxime axetil and water-soluble excipient in the solvent or solvents may be prepared either by dissolving the cefuroxime axetil and water-soluble excipient into solvents separately and then mixing the two solutions together, or by directly adding the cefuroxime axetil and water-soluble excipient to the solvent or mixture of solvents and mixing until a clear solution is formed.

After the solution of the cefuroxime axetil and water-soluble excipient in the solvent or solvents is prepared, it is necessary to then remove the solvent or solvents to obtain a dry co-precipitate.

This may be done, for example, by evaporating the solvent or solvents in a spray drying or roller drying process, or by evaporating the solvent or solvents under vacuum.

The dried co-precipitate comprising cefuroxime axetil and the water-soluble excipient will then be further processed into a tablet.

This may be done by mixing the co-precipitate with other excipients and then processing the mixed powder into tablets on a tablet press. The other excipients will preferably include both a disintegrant and a lubricant.

The disintegrant is an ingredient which absorbs water and swells to cause the tablet to disintegrate when the tablet is immersed in gastro-intestinal fluid. Preferred disintegrants are water-insoluble cross-linked polymers, including, for example, croscarmellose sodium, sodium starch glycolate, and crospovidone.

A lubricant is needed to prevent sticking of the powder to the tooling in the tableting process. Preferred lubricants are stearic acid and metallic stearates, such as magnesium stearate.

It will be understood that, as an alternative to preparing the dry co-precipitate by evaporation of solvents and then mixing the co-precipitate with other excipients in a subsequent step, the two steps may be done together. This may be done, for example, by spraying the solution of cefuroxime axetil and the water-soluble excipient onto other excipients in a fluidized bed drying system.

The invention will be further illustrated by the following examples, which are intended to be illustrative but not limiting of the scope of the invention.

EXAMPLE 1

2000 g of acetone and 200 g of methanol were placed in a beaker. While stirring, 500 g of pure crystalline cefuroxime axetil was slowly added, and stirring was continued for about 5 minutes, until the cefuroxime axetil was fully dissolved. Stirring was continued and 50 g of hydroxy propyl cellulose was then added. Stirring was continued for another several minutes, until the hydroxy propyl cellulose was fully dissolved. The solution was then spray-dried to obtain a co-precipitate comprising 1 part hydroxpropyl cellulose to 10 parts cefuroxime axetil.

EXAMPLE 2

The following were mixed together:

| | |
|---|---|
| co-precipitate from example 1 | 134.2 g |
| croscarmellose sodium | 44.0 g |
| magnesium stearate | 1.0 g |
| colloidal silicon dioxide | 0.8 g |
| Total | 180.0 g |

The mixed powder was compacted into slugs on a tablet press. The slugs were then ground into granules, and the granules were recompressed on a tablet press into tablets of weight 900 mg.

In view of the proportions of ingredients as aforesaid, each tablet contained 671 mg of co-precipitate, which in turn contained 610 mg of cefuroxime axetil, which in turn is equivalent to about 500 mg of cefuroxime.

The tablets were tested for disintegration time using the method set out in the United States Pharmacopoeia, 23rd edition, page 1791. The disintegration time was over 30 minutes.

The tablets were also tested for dissolution as set out in the United States Pharmacopoeia, 23rd edition, page 316. The result was about 65% in 20 minutes and 90% in 60 minutes.

The dissolution specifications for cefuroxime axetil tablets on the said page 316 are 65% in 20 minutes and 80% in 60 minutes. The tablets of this example were thus found to comply with this specification, despite the relatively slow disintegration.

The dissolution specifications in the United States Pharmacopoeia are designed to ensure that tablets meeting the specifications will exhibit acceptable bioavailability.

EXAMPLE 3

2000 g of acetone and 200 g of methanol were placed in a beaker. While stirring, 500 g of pure crystalline cefuroxime axetil was slowly added, and stirring was continued for about 5 minutes, until the cefuroxime axetil was fully dissolved. Stirring was continued and 50 g of povidone was then added. Stirring was continued for another several minutes, until the povidone was fully dissolved. The solution was then spray-dried to obtain a co-precipitate comprising 1 part povidone to 10 parts cefuroxime axetil.

EXAMPLE 4

The following were mixed together:

| | |
|---|---|
| co-precipitate from example 3 | 132.0 g |
| croscarmellose sodium | 43.6 g |
| magnesium stearate | 1.0 g |
| colloidal silicon dioxide | 0.8 g |
| Total | 177.4 g |

The mixed powder was compacted into slugs on a tablet press. The slugs were then ground into granules, and the granules were recompressed on a tablet press into tablets of weight 900 mg.

Again, in view of the proportions of ingredients as aforesaid, each tablet contained 670 mg of co-precipitate, which in turn contained 609 mg of cefuroxime axetil, which in turn is equivalent to about 500 mg of cefuroxime.

The tablets were tested for disintegration time using the method set out in the United States Pharmacopoeia, 23rd edition, page 1791. The disintegration time was about 10 minutes.

The tablets were also tested for dissolution as set out in the United States Pharmacopoeia, 23rd edition, page 316. The result was over 80% in 20 minutes and over 90% in 60 minutes.

The tablets of this example thus exhibited dissolution substantially faster than required by the United States Pharmacopoeia, again despite the fact that disintegration was not immediate.

**Claims**

1. A co-precipitate comprising cefuroxime axetil and a water-soluble excipient.

2. A co-precipitate as in claim 1 comprising from about 40% to about 98% by weight cefuroxime axetil and from about 2% to about 60% by weight water- soluble excipient.

3. A co-precipitate as in claim 1 comprising from about 75% to about 95% by weight cefuroxime axetil and from about 5% to about 25% by weight water-soluble excipient.

4. A co-precipitate as in claim 1 comprising about 90% by weight cefuroxime axetil and about 10% by weight water-soluble excipient.

5. A co-precipitate as in any one of claims 1 to 4 wherein the water-soluble excipient is selected from the group consisting of povidone, hydroxy propyl cellulose, methycellulose, lactose, mannitol and sorbitol.

6. A process of production of a co-precipitate of any one of claims 1 to 5 which comprises:-

   - dissolving the cefuroxime axetil and water-soluble excipient in a solvent or a mixture of solvents; and
   - evaporating the solvent or solvents.

7. A process as in claim 6 wherein acetone is used as solvent.

8. A process as in claim 6 wherein the solvent or solvents are evaporated by spray-drying.

9. A pharmaceutical tablet comprising a co-precipitate according to any one of claims 1 to 5.

10. A pharmaceutical tablet as in claim 9 further comprising a disintegrant.

11. A pharmaceutical tablet as in claim 10 wherein the disintegrant is a water-insoluble cross-linked polymer.

12. A pharmaceutical tablet as in claim 10 wherein the disintegrant is selected from the group consisting of croscarmellose sodium, sodium starch glycolate and crospovidone.

13. A pharmaceutical tablet as in claim 10 further comprising a lubricant.

14. A pharmaceutical tablet as in claim 13 wherein the lubricant is stearic acid or a metallic stearate.

**Patentansprüche**

1. Copräzipitat, umfassend Cefuroximaxetil und einen wasserlöslichen Träger.

2. Copräzipitat nach Anspruch 1, umfassend etwa 40 Gew.-% bis etwa 98 Gew.-% Cefuroximaxetil und etwa 2 Gew.-% bis etwa 60 Gew.-% des wasserlöslichen Trägers.

3. Copräzipitat nach Anspruch 1, umfassend etwa 75 Gew.-% bis etwa 95 Gew.-% Cefuroximaxetil und etwa 5% bis etwa 25 Gew.-% des wasserlöslichen Trägers.

4. Copräzipitat nach Anspruch 1, umfassend etwa 90 Gew.-% Cefuroximaxetil und etwa 10 Gew.-% des wasserlöslichen Trägers.

5. Copräzipitat nach einem der Ansprüche 1 bis 4, wobei der wasserlösliche Träger ausgewählt ist aus der Gruppe, bestehend aus Povidon, Hydroxypropylcellulose, Methylcellulose, Lactose, Mannit und Sorbit.

6. Verfahren zur Herstellung eines Copräzipitats nach einem der Ansprüche 1 bis 5, welches

   das Lösen von Cefuroximaxetil und des wasserlöslichen Trägers in einem Lösungsmittel oder einem Lösungsmittelgemisch und
   das Abdampfen des Lösungsmittels oder der Lösungsmittel umfaßt.

7. Verfahren nach Anspruch 6, worin Aceton als Lösungsmittel verwendet wird.

8. Verfahren nach Anspruch 6, wobei das Lösungsmittel oder die Lösungsmittel durch Sprühtrocknung verdampft werden.

9. Pharmazeutische Tablette, umfassend ein Copräzipitat nach einem der Ansprüche 1 bis 5.

**10.** Pharmazeutische Tablette nach Anspruch 9, weiter umfassend ein Abbaumittel.

**11.** Pharmazeutische Tablette nach Anspruch 10, wobei das Abbaumittel ein wasserunlösliches quervernetztes Polymer ist.

**12.** Pharmazeutische Tablette nach Anspruch 10, wobei das Abbaumittel ausgewählt ist aus der Gruppe, bestehend aus Natriumcroscarmellose, Natriumstärkeglykolat und Crospovidon.

**13.** Pharmazeutische Tablette nach Anspruch 10, weiter umfassend ein Gleitmittel.

**14.** Pharmazeutische Tablette nach Anspruch 13, wobei das Gleitmittel Stearinsäure oder ein Metallstearat ist.

**Revendications**

**1.** Co-précipité contenant du céfuroxime axétil et un excipient soluble dans l'eau.

**2.** Co-précipité selon la revendication 1, contenant environ 40% à environ 98% en poids de céfuroxime axétil et environ 2% à environ 60% en poids d'excipient soluble dans l'eau.

**3.** Co-précipité selon la revendication 1, contenant environ 75% à environ 95% en poids de céfuroxime axétil et environ 5% à environ 25% en poids d'excipient soluble dans l'eau.

**4.** Co-précipité selon la revendication 1, contenant environ 90% en poids de céfuroxime axétil et environ 10% en poids d'excipient soluble dans l'eau.

**5.** Co-précipité selon l'une quelconque des revendications 1 à 4, dans lequel l'excipient soluble dans l'eau est choisi dans le groupe constitué par la povidone, l'hydroxypropylcellulose, la méthylcellulose, le lactose, le mannitol et le sorbitol.

**6.** Procédé de préparation d'un co-précipité selon l'une quelconque des revendications 1 à 5, qui comprend:

- la dissolution du céfuroxime axétil et de l'excipient soluble dans l'eau dans un solvant ou un mélange de solvants; et
- l'évaporation du ou des solvants.

**7.** Procédé selon la revendication 6, dans lequel l'acétone est utilisée comme solvant.

**8.** Procédé selon la revendication 6, dans lequel le ou les solvants sont évaporés par séchage par pulvérisation.

**9.** Comprimé pharmaceutique contenant un co-précipité selon l'une quelconque des revendications 1 à 5.

**10.** Comprimé pharmaceutique selon la revendication 9, contenant en outre un désintégrant.

**11.** Comprimé pharmaceutique selon la revendication 10, dans lequel le désintégrant est un polymère réticulé insoluble dans l'eau.

**12.** Comprimé pharmaceutique selon la revendication 10, dans lequel le désintégrant est choisi dans le groupe constitué par la croscarmellose sodique, le glycolate d'amidon sodique et la crospovidone.

**13.** Comprimé pharmaceutique selon la revendication 10, contenant en outre un lubrifiant.

**14.** Comprimé pharmaceutique selon la revendication 13, dans lequel le lubrifiant est l'acide stéarique ou un stéarate métallique.